# EUROPÄISCHE PATENTANMELDUNG

(11) **EP 3 205 348 A1**
(43) Veröffentlichungstag der Anmeldung: **16.08.2017**
(21) Anmeldenummer: 16155735.0
(22) Anmeldetag: 15.02.2016
(51) Int. Cl.: A61K 38/16, A61K 36/185, C07K 14/42, A61P 35/00, C07K 14/00

(54) **ARZNEIMITTEL ENTHALTEND REKOMBINANTE MISTELLEKTINE ZUR BEHANDLUNG VON HIRNTUMOREN**

(71) Anmelder: Melema Pharma GmbH, 20148 Hamburg (DE)
(72) Erfinder: LENTZEN, Hans, 51503 Rösrath (DE)
(74) Vertreter: Simandi, Claus

(57) **Zusammenfassung**

Die Erfindung betrifft ein Arzneimittel und / oder pharmazeutische Zusammensetzung enthaltend rekombinante Mistellektine zur Behandlung von Hirntumoren, insbesondere primäre Hirntumore, Gliome, Glioblastome, Meningeome und Hypophysenadenome, und dessen Verwendung.

## Beschreibung

Die Erfindung betrifft ein Arzneimittel und / oder pharmazeutische Zusammensetzung enthaltend rekombinante Mistellektine zur Behandlung von Hirntumoren, insbesondere primäre Hirntumore, Gliome, Glioblastome, Meningeome und Hypophysenadenome, und dessen Verwendung.

Primäre Hirntumore, wie Gliome, Glioblastome, Meningeome und Hypophysenadenome, gehen von Neuroepithel, Ganglienzellen, Meningen, Nervenscheiden, allgemeinem Nervenstützgewebe bzw. Neuroglia und Hypophyse oder ektopen intrakraniellen Geweben (Keimzell- oder Fehlbildungstumoren) aus und deren Ursache sind vor allem in genetischen und hormonalen Faktoren, onkogenen Viren und exogenen Karzinogenen zu sehen. Es handelt sich um im Gehirn lokalisierte, echte Tumore des zentralen Nervensystems (ZNS) unterschiedlicher Differenzierung als auch Unterarten, wie: astrozytäre Tumore, Oligodendrogliom, Mischgliom (Oligoastrozytom), Ependymom, Tumore des Plexus choroideus, Retinoblastom, u.a..

Die WHO-Grad-Einteilung erfolgt entsprechend der Dignität: Grad I (gutartig / benigne), Grad II (semibenigne; postoperative Überlebenszeit 3-5 Jahre), Grad III (semimaligne; postoperative Überlebenszeit 2-3 Jahre), Grad IV (maligne; postoperative Überlebenszeit 6-15 Mon.); Häufigkeit: Anteil der gesamten bzw. primären Hirntumore an allen Tumorerkrankungen: 7-9 % (Kleihues, P., Louis, D. N., Scheithauer, B. W., Rorke, L. B., Reifenberger, G., Burger, P. C., and Cavenee, W. K. (2002) The WHO classification of tumors of the nervous system. J. Neuropathol. Exp. Neurol. 3, 215-225).

Ein Gliom wird histologisch in (Riesenzell-)(Oligo-) Astrozytomen, Oligodendrogliom, Mischgliome, Glioblastom charakterisiert und differenziert je nach Wachstum, wie isomorph, anaplastisch, pilozystisch u.a..
Ferner können Untergruppen von Gliomen anhand des Verlustes der Heterozygotie ("loss of heterocygosity") prognostiziert werden (Smith, J. S., and Jenkins, R. B. (2000) Genetic alterations in adult diffuse glioma: occurrence, significance, and prognostic implications. Front Biosci. 5, 213-231), die zum Verlust von Tumorsuppressorgenen führen (Tews, B., Felsberg, J., Hartmann, C., Kunitz, A., Hahn, M., Toedt, G., Neben, K., Hummerich, L., von Deimling, A., Reifenberger, G., and Lichter, P. (2006) Identification of novel oligodendroglioma-associated candidate tumor suppressor genes in 1p36 and 19q13 using microarray-based expression profiling. Int J Cancer. 119, 792-800).

Insbesondere Glioblastome (GBM) zählen zu den bösartigsten hirneigenen Tumoren. Das mediane Überleben von GBM Patienten liegt, selbst bei besten Therapievoraussetzungen, bei nur etwa 12-15 Monaten. Natürliche Killer (NK) Zellen als Bestandteile des angeborenen Immunsystems spielen eine wichtige Rolle bei der Zerstörung von Krebszellen. GBM Zellen entwickeln Strategien, diesem Killing zu entgehen, indem sie Proteine, die für die Interaktion mit NK-Zellen notwendig sind, die sogenannten Danger/Stranger Proteine major histocompatibility complex (MHC), MHC class I polypeptide-related sequence (MIC)-A and -B oder UL16 binding proteins (ULBP) 1, 2, 3., downregulieren und zwar mittels TGF-β mediierter Immunsuppression. Hierzu ist die Überexpression von TGF-β ein wesentliches Merkmal von GBM und hohe Konzentrationen von TGF-β können bei Gliom-Patienten in der Ceresbrospinal-Flüssigkeit nachgewiesen werden, welche mit dem Wachstum des Tumors korreliert (Kjellman C, Olofsson SP, Hansson O et al: Expression of TGF-beta isoforms, TGF-beta receptors, and SMAD molecules at different stages of human glioma. Int J Cancer 2000; 89: 251-258). Darüber hinaus ist TGF-β verantwortlich für die down-Regulierung der MHC-Expression, Steigerung der Differenzierung von naiven Zellen in T-reg Zellen, Blockierung dendritischer Zellmaturation, und Zelltodinduzierung von NK- und T-Zellen (Eisele G, Wischhusen J, Mittelbronn M et al.: TGF-beta and metalloproteinases differentially suppress NKG2D ligand surface expression on malignant glioma cells. Brain 2006; 129: 2416-2425, Platten M, Wick W, Weller M: Malignant glioma biology: role for TGF-beta in growth, motility, angiogenesis, and immune escape. Microsc Res Tech2001; 52: 401-410).

Es besteht daher ein großes Bedürfnis Arzneimittel zur Behandlung und Prophylaxe von Hirntumor, insbesondere primäre Hirntumore, wie Gliome, Glioblastome, Meningeome und Hypophysenadenome bereitzustellen.

Mistelextrakte werden seit Jahrhunderten therapeutisch genutzt. Insbesondere in der Krebstherapie sind Mistelpräparate mit unterschiedlichem Erfolg eingesetzt worden (Bocci V 1993 J Biol Regulators and Homeostatic Agents 7(1): 1 - 6; Gabius H-J, Gabius S, Joshi S S et al. 1993 Planta Med 60: 2 - 7; Gabius H-J & Gabius S 1994 PZ 139: 9 - 16; Ganguly C & Das S 1994 Chemotherapy 40: 272 - 278, Hajto T, Hostanska K, Gabius H_J 1989 Cancer Res 49: 4803 - 4808, Hajto T, Hostanska K, Frei K et al. 1990 Cancer Res. 50: 3322 - 3326). Es zeigte sich, dass die therapeutischen Effekte insbesondere durch sogenannte Mistellektine (Viscumine, Viscum album Agglutinine, VAA) vermittelt werden. Den Mistellektinen wird dabei neben einer zytotoxischen Wirkung auch eine unspezifische Immunstimulation zugesprochen, deren positive Effekte zur Therapie von Tumorpatienten genutzt werden. Verschiedene Untersuchungen mit Mistellektinen in vitro (Hajto et al., 1990 (supra); Männel D N, Becker H, Gundt A et al. 1991 Cancer Immunol Immunother 33: 177 - 182; Beuth J, Ko K L, Tunggal L et al. 1993 Drug Res 43: 166 - 169) und in vivo (Hajto T 1986 Oncology 43 suppl 1: 51 - 65; Hajto et al., 1989 (supra), Beuth J, Ko H L, Gabius H-J et al. 1991 In Vivo 5: 29 - 32; Beuth J, Ko H L, Gabius H-J et al. 1992 J Clin Invest 70: 658 - 661), sowie klinische Studien (Beuth et al., 1992 (supra)) zeigten eine erhöhte Freisetzung von inflammmatorischen Zytokinen (TNF-alpha, IL-1, IL-6) und eine Aktivierung von zellulären Komponenten des Immunsystems (TH - Zellen, NK-Zellen, B- und T-Lymphozyten) (Braedel-Ruoff S: Immunomodulatory effects of Viscum album extracts on natural killer cells: review of clinical trials. Forsch Komplementmed 2010; 17: 63-73, Gren A : Effects of Iscador preparations on the reactivity of mouse immune system. Neuro Endocrinol Lett 2009; 30: 530-534, Lee CH, Kim JK, Kim HY et al.: Immunomodulating effects of Korean mistletoe lectin in vitro and in vivo. Int Immunopharmacol 2009; 9: 1555-1561, Nikolai G, Friedl P, Werner M et al: Effect of a mistletoe extract (Iscador QuFrF) on viability and migratory behavior of human peripheral CD4+ and CD8+ T lymphocytes in three-dimensional collagen lattices. In Vitro Cell Dev Biol Anim 1997; 33: 710-716).

Durch Analysen des Mistelextraktes konnten bisher drei Mistellektine (ML-I, ML-II, ML-III) mit unterschiedlichen Molekulargewichten und Zuckerbindungsspezifitäten identifiziert werden. Es konnte gezeigt werden, dass der immunstimulierende Effekt des Mistelextrakts auf das ML-I zurückzuführen ist. Das ML-I-Lektin besteht aus jeweils zwei glykosylierten A- und B-Ketten (MLA bzw. MLB). Die A-Kette ist für eine enzymatische Inaktivierung von Ribosomen (Endo Y, Tsurugi K & Franz H 1988 FEBS Lett 231: 378 - 380) verantwortlich, während die B-Kette bei der Carbohydratbindung beteiligt ist. Die beiden Ketten sind durch Disulfidbrücken miteinander verknüpft. Die resultierenden Mistellektin-Monomere können sich unter Ausbildung von nicht kovalenten Bindungen zu Dimeren zusammenlagern.

Es ist möglich, das biologisch aktive Mistellektin vorteilhaft rekombinant herzustellen. EP 0751221 beschreibt die Reindarstellung von Mistellektin-Polypeptiden als strukturell homogene Substanz, wobei ausgehend von den Gensequenzen des Mistellektins rekombinante, hochreine Einzelketten (A-Kette, B-Kette) hergestellt werden, die in vitro reassoziiert werden können und so ein rekombinantes Mistellektin-Holoprotein ergeben, das proteinchemisch, enzymatisch und strukturell homogen ist, sogenanntes Aviscuminum. Gemäß EP 0751221 ist das rekombinante Mistellektin-Polypeptid sowohl als Holoprotein, als Teilkette und in Form von Subfragmenten für therapeutische Zwecke geeignet und erfindungsgemäß umfasst.

Weiterhin beschreibt WO2012104355A1 die antivirale Wirkung von rekombinanten Mistellektinen. WO2012136857A1 offenbart die Behandlung von Hautkrebs, insbesondere eines malignen Melanoms auch in der Form eines metastasierenden Tumors mittels rekombinanten Mistellektinen.

Bisher wurden rekombinante Mistellektine vorteilhaft in der Behandlung von Tumorerkrankungen eingesetzt. Die Verwendung von rekombinanten Mistellektinen zur Behandlung von Hirntumor, insbesondere primäre Hirntumore, wie Gliome, Glioblastome, Meningeome und Hypophysenadenome ist jedoch nicht im Stand der Technik beschrieben.

Im Stand der Technik beschreibt Podlech et al (Podlech O, Harter PN, Mittelbronn M et al.: Fermented mistletoe extract as a multimodal antitumoral agent in gliomas. Evid Based Complement Alternat Med 2012: 501796) die Verwendung von ISCADOR - ein fermentativ gewonnener Mistellextrakt - als Wachstumsinhibitor für GBM und schließt auf die antitumorale Eignung von ISCADOR zur Behandlung von GBM.

Lenartz (Lenartz et al., Immunoprotective Activity of the Galactoside-Specific lectin from misletoe after Tumor Destructive Theraly in Glioma Patients, Anticancer Research 16: 3799-3802 (1996)) berichtet über einen Mistellextrakt (ML-1) zur Behandlung von Glioma Patienten, wobei die Mistelextrakte eine spezifische Glykolisierung aufweisen.

Die im Stand der Technik beschriebenen pflanzlichen Mistelllektine - seien sie aus fermentativ oder nicht fermentativ hergestellten Mistelextrakten gewonnen - sind inhomogen (Soler MH, Stoeva S, Schwamborn C et al. 1996 FEBS Letter 399: 153-157, Soler HS, Stoeva S, Voelter W 1998 Biochem Biophys Res Comm 246: 596-601) und untereinander in der Wirkung uneinheitlich bis verschieden (EP 1051495 B1), und nicht per se als Wirkstoff oder als Immunmodulator wirksam. Von daher ist z.B. das aus der koreanischen Mistel *(Viscum album colaratum)* gewonnene Mistellektin zwar den RIP II Proteinen zuzuordnen, weist jedoch starke strukturelle Unterschiede in der Struktur und Konformation gegenüber den hier in Rede stehenden rekombinanten Mistellektinen auf (Kang TB, Song SK, Yoon TJ et al. 2007 J Biochem Mol Biol 40(6): 959-965). Besonders nachteilig ist, dass keine exakte Dosiseinstellung möglich ist und aus pflanzlichen fermentativ oder nicht-fermentativ hergestellten Extrakten gewonnene Mistellektine Verunreinigungen aufweisen. Weiterhin zeigen die aus pflanzlichen Extrakten - fermentativ oder nicht-fermentativ hergestellt - gewonnenen Mistellektine Unterschiede in der Glycolisierung, die die Wirksamkeit beeinflussen (insbesondere Kinetik, etc.). Abgesehen hiervon ergibt die Herstellung jeder neuen Mistelextrakt-Charge ein zu der vorherigen Charge nicht identisches Produkt mit unterschiedlichen Gehalten seiner Inhaltsstoffe inkl. der glykosylierten Mistellektine.

Die erfindungsgemäßen rekombinanten Mistellektine weisen vorteilhaft keine solche Glycolisierung auf, sind absolut rein und sind reproduzierbar herzustellen.

Überraschenderweise konnte nunmehr gezeigt werden, dass rekombinante Mistellektine nicht nur die besagten Vorteile, wie bessere Reproduzierbarkeit, Homogenität und einstellbare Dosierung aufweisen, sondern gegenüber einem pflanzlichen oder fermentativen Mistelextrakt aus dem Stand der Technik eine verbesserte NK-Zell-Zytotoxizität über den NK Zell Oberflächenmarker NKG2D hervorruft und daher für die Behandlung von Hirntumoren, Gliomen, Glioblastomen, Meningeomen und Hypophysenadenomen besonders geeignet sind.

Natürliche Killer (NK) Zellen als Bestandteile des angeborenen Immunsystems spielen eine wichtige Rolle bei der Zerstörung von Krebszellen. Um das Zerstören der Tumorzellen durch NK-Zellen zu erreichen ist das Andocken von NK-Zellen an Tumorzellen erforderlich. Dabei spielen der NKG2D-Rezeptor auf NK-Zellen und zur Aktivierung notwendige Oberflächenproteine (NKp30, NKp44, NKp46) eine wichtige Rolle. In den Beispielen wird die Wirkung der rekombinanten Mistellektine mit der Wirkung des fermentierten Mistelextraktes ISCADOR Q auf die Interaktion von NK-Zellen mit der GBM Zelle LNT-229-Luc verglichen.

Die Aufgabe der vorliegenden Erfindung besteht daher darin, ein Arzneimittel und pharmazeutisches Mittel zur Behandlung von Hirntumor, insbesondere primäre Hirntumore, wie Gliome, Glioblastome, Meningeome und Hypophysenadenome bereitzustellen.

Die Aufgabe wird durch die Bereitstellung eines Arzneimittels sowie einer pharmazeutischen Zusammensetzung gelöst, wobei diese rekombinanten Mistellektine zur Behandlung von Hirntumor, insbesondere primäre Hirntumore, wie Gliome, Glioblastome, Meningeome und Hypophysenadenome enthalten.

Vorzugsweise umfasst das erfindungsgemäße Arzneimittel die Mistellektin-A-Kette (MLA) beziehungsweise die Mistellektin-B-Kette (MLB), jeweils einzeln oder zusammen, auch in Form von Dimeren (siehe z.B. EP 0 751 221 oder EP 1 051 495).

Das rekombinante Mistellektin-Polypeptid der Mistellektin-A Kette umfasst die folgenden Sequenzen: SEQ ID No. 1 - 3, einschließlich deren Isoformen oder ein funktionelles Fragment davon.

Das rekombinante Mistellektin-Polypeptid der Mistellektin-B Kette umfasst die folgenden Sequenzen SEQ ID No. 4 - 12, einschließlich deren Isoformen oder ein funktionelles Fragment davon.

### (nachstehend allesamt "rekombinante Mistellektine")

Weiterhin bevorzugt ist ein erfindungsgemäßes rekombinantes Mistellektin ein Heterodimer bestehend aus den Sequenzen SEQ ID No. 1 und SEQ ID No. 4, siehe z.B. EP 0 751 221 (sogenanntes Aviscuminum).

Der Begriff "funktionelles Fragment" definiert im Zusammenhang mit dieser Erfindung Fragmente der genannten Polypeptide, welche die gleiche biologische Funktion besitzen, wie das mit der jeweiligen Aminosäuresequenz oben dargestellte Polypeptid.

Der Begriff "gleiche biologische Funktion" beschreibt in diesem Zusammenhang beispielsweise, dass Fragmente oder Derivate der Polypeptide die gleichen Signale in einer Zelle induzieren, wie die genannten Peptide. Beispiele für Fragmente sind Peptiddomänen mit definierten Funktionen. Die "gleiche biologische Funktion" umfasst auch die Zytotoxizität, Immunstimmulation (sowohl des nativen, als auch des adaptiven Immunsysterns), Stimulation der Freisetzung von Zytokinen, Antigenität, die Induktion der Expression oder die Aktivierung von Oberflächenmarkern, die Induktion von Apoptose oder Endorphin-Stimulation.

Unter "biologischer Aktivität des rekombinanten Mistellektins" wird hier jede biologische Aktivität aus dem Spektrum der gesamten biologischen Aktivitäten des rekombinanten Mistellektins verstanden. Eine derartige Funktion ist z.B. die pharmakologische Wirkung des rekombinanten Mistellektins.

Untersuchungen von ML-I-Monomeren ergaben 25 verschiedene Isoformen, die auf unterschiedliche Kombinationen verschiedener A- und B-Ketten sowie unterschiedliche Glykosylierungszustände der Ketten zurückzuführen sind.

Für die vorliegende Erfindung kommt daher ein Mistellektin-Polypeptid oder ein Fragment davon, welches die Sequenzvariabilität der verschiedenen MLA- und MLB-Ketten umfasst, zu den Sequenzen SEQ ID No. 1 - 12 erfindungsgemäß in Betracht.

Vorzugsweise enthält das erfindungsgemäße Arzneimittel ein rekombinantes Mistellektin-Polypeptid mit den Sequenzen SEQ ID No. 1 - 12 oder einem funktionellen Fragment davon oder eine beliebige Kombination davon.

Bevorzugt ist weiterhin, dass die Verwendung von erfindungsgemäßen rekombinanten Mistellektinen bei solchen Patientenpopulationen zum Tragen kommt, die mittels einer Standardtherapie nicht auf Tumorpräparate ansprechen, bzw. Nicht-Responder oder Therapieversager umfassen.

Daher umfasst die Erfindung solche Patienten oder Patientenpopulationen von Nicht-Respondern und Therapieversagern, zur Behandlung von Hirntumor, insbesondere primäre Hirntumore, wie Gliome, Glioblastome, Meningeome und Hypophysenadenome, bei denen eine Standardtumortherapie nicht erfolgreich ist.

Der Begriff "Hirntumor" umfasst erfindungsgemäß primäre Hirntumore, wie Gliome, Meningeome und Hypophysenadenome, ausgehend von Neuroepithel, Ganglienzellen, Meningen, Nervenscheiden, allgemeinem Nervenstützgewebe bzw. Neuroglia und Hypophyse oder ektopen intrakraniellen Geweben (Keimzellod. Fehlbildungstumoren) und deren Ursache vor allem in genetischen und hormonalen Faktoren, onkogene Viren und exogene Karzinogene zu sehen sind.

Eine bevorzugte erfindungsgemäße Ausführungsform ist jedoch die Behandlung von Gliomen (siehe z.B. Beschreibung der Indikationen im Pschyrembel ®, 266. Auflage 2014, De Gruyter Verlag, Berlin).

Die Erfindung betrifft außerdem ein Arzneimittel zur Behandlung von Hirntumor, insbesondere primäre Hirntumore, wie Gliome, Glioblastome, Meningeome und Hypophysenadenome, welches das rekombinante Mistellektin-Polypeptid, gegebenenfalls zusammen mit einem pharmazeutisch verträglichen Träger enthält unter Ausbildung einer pharmazeutischen Zusammensetzung. Beispiele für besonders geeignete pharmakologisch verträgliche Träger sind dem Fachmann auf dem Gebiet der Tumorheilkunde bekannt und umfassen gepufferte Kochsalzlösungen, Wasser, u.a., verschiedene Arten von Detergenzien, sterile Lösungen, etc. Arzneimittel, die solche Träger umfassen, können mittels bekannter konventioneller Methoden formuliert werden. Diese Arzneimittel können einem Individuum in einer geeigneten Dosis verabreicht werden. Die Verabreichung kann lokal, oral oder parenteral erfolgen, z.B. intravenös, intraperitoneal, subcutan, intramuskulär, lokal, intranasal, intrabronchial oder intradermal, oder über einen Katheter an einer Stelle in einer Arterie. Die Art der Dosierung wird vom behandelnden Arzt entsprechend den klinischen Faktoren bestimmt. Es ist dem Fachmann bekannt, dass die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden.

Die pharmazeutische Zusammensetzung, welche die erfindungsgemäßen rekombinanten Mistellektin-Polypeptide umfasst, kann lokal oder systemisch verabreicht werden.

Als vorteilhaft hat sich eine Dosierung der erfindungsgemäßen Mistellektine für die humane Anwendung von 2 - 10 ng/kg (Körpergewicht) erwiesen. Besonders vorteilhaft ist die Dosierung einem Bereich von 3 - 7 ng/kg. Vorzugsweise beträgt die verabreichte Menge 5 ng/kg Körpergewicht. Die bevorzugte nicht auf das Körpergewicht bezogene humane Dosierung beträgt 350 ng.

Das erfindungsgemäße Arzneimittel wird über eine Zeitdauer von mindestens 8 Wochen mit einer Frequenz von 1 x täglich bis 1 x pro Woche appliziert. Vorzugsweise wird das Arzneimittel 2 bis 3 x pro Woche verabreicht, besonders bevorzugt ist 2 x pro Woche.

Daher betrifft die Erfindung ein Verfahren zur Dosierung der erfindungsgemäßen rekombinanten Mistellektine oder des erfindungsgemäßen Arzneimittels, wobei die Dosierung 2 bis 10 ng/kg (Körpergewicht) beträgt. Insbesondere betrifft die Erfindung ein Verfahren zur Dosierung der erfindungsgemäßen rekombinanten Mistellektine oder des erfindungsgemäßen Arzneimittels, wobei die Dosis 200 - 500 ng, inbesondere 350 ng beträgt und mindestens 1 x pro Woche dem Patienten verabreicht wird.

Nachfolgende Beispiele und Figuren dienen zur Erläuterung der Erfindung, ohne jedoch die Erfindung auf diese Beispiele zu beschränken.

### Beispiele und Figuren:

### Beispiel 1 einer Zusammensetzung des Arzneimittels

Lösung zur Injektion: 1 mL Ampulle mit 0,5 mL bis 1,0 mL Injektionslösung

| | |
|---|---|
| Aviscuminum | 200 - 500 ng |
| Natriummonohydrogenphosphat Dihydrat | 2,8 mg - 5,6 mg |
| Natriumdihydrogenphosphat Dihydrat | 0,078 mg - 0,155 mg |
| Natriumchlorid | 3,3 mg - 6,7 mg |
| Polyoxyethylen sorbitan ester (Polysorbat) | 0, 1 mg |
| Glutaminsäure | 0,1 mg |
| Wasser zur Injektion | ad 0,5 ml bis ad 1,0 mL |

### Beispiel 2 einer Zusammensetzung des Arzneimittels

Pulver zur Herstellung einer Lösung zur Injektion, 2R Glasvial mit

| | |
|---|---|
| Aviscuminum | 200 - 500 ng |
| Trehalose | 40,0 mg |
| Natriumchlorid | 1,0 mg |
| Tris(hydroxymethyl)aminomethan (TRIS) | 0,6 mg |
| Polyoxyethylen sorbitan ester (Polysorbat) | 0,1 mg |

Salzsäure zur Einstellung des pH Werts
zur Verabreichung wird das Pulver mit 0,5 mL oder 1,0 mL Wasser zur Injektion gelöst.

### Beispiel 3:

Zur Methodik: LNT-229-Luc Zellen wachsen in DMEM (Sigma, Taufkirchen, Germany) Medium mit 10% fetalem Kälberserum und Penicillin/ Streptomycin in einer befeuchteten Atmosphäre angereichert mit 5% CO2.

Zur Isolierung der NK-Zellen werden PBMC aus dem Blut gesunder Donoren gewonnen und mit bestrahlten RPMI 8866 Feeder-Zellen (ATCC, USA) in RPMI Medium (Sigma, Taufkirchen, Germany) kokultiviert, um polyklonale NK Zellpopulationen zu erhalten. Die NK-Zellen werden mit Hilfe eines NK-Zell Isolationskits (Miltenyi Biotec, Bergisch Gladbach, Germany) >98% aufgereinigt. Ihre lytische Aktivität gegenüber GBM Zellen wird über die Luciferase Aktivitätsmessung bestimmt. Für die Inaktivierung der NK-Zellaktivität werden die NK-Zellen mit Antikörpern gegen NKG2D (BioLegend, Fell, Germany) 30 Minuten vor ihrer 4 stündigen Kokultivierung (Effektor-/Targetzelle 20:1) mit den GBM-Zellen vorinkubiert. Die GBM Zellen werden ihrerseits vor der Kokultivierung mit den NK-Zellen 24 h mit Aviscuminum bzw. ISCADOR Q vorbehandelt und vor ihrer Verwendung in der Kokultur gewaschen.

Ergebnis: Aviscuminum beeinflusst die Wirkung von NK-Zellen auf die GBM Zelle, indem es die Interaktion ihres Rezeptors NKG2D mit der Zielzelle verstärkt.

Die Blockade des NKG2D-Rezeptors durch Antikörper führt erwartungsgemäß zu einer Aufhebung der NK-Zell vermittelten Lyse um 52% in der Kontrollgruppe (Medium); aviscuminum antagonisiert die Wirkung des Antikörpers teilweise: Die NK-Zell vermittelte Lyse wird durch den Antikörper demnach nur um 38% aufgehoben. Ein Vergleich mit dem fermentierten Mistelextrakt ISCADOR Q war unter diesen Bedingungen nicht möglich, da bei gleicher auf Mistellektin bezogener Konzentration von 8 ng/ml die GBM-Zellen ohne Einfluss von NK-Zellen deutlich stärker als durch die Kontrolle vermittelt lysiert werden. Offenbar sind weitere Inhaltsstoffe des fermentierten Extraktes direkt zytotoxisch (Tabelle).

| GBM Zelllyse | | | |
|---|---|---|---|
| | Ohne AK Vorbehandlung | anti-NKG2D Vorbehandlung | |
| Medium | 67% | 32,1% (Hemmung: d. Lyse um 52,1%) | P<0,05 |
| Aviscuminum | 68,4% | 42,6% (Hemmung d. Lyse um 37,7%) | P<0,05 |
| Iscador Q | 83,2% | 61,6% | not significant |

Lt. Podlech wird ISCADOR Q (200 mg) für Inkubationsexperimente 2000fach verdünnt (100 migrogramm/ml). Der Extrakt enthält u.a. verschiedene Mistellektine, deren Gehalt in der verwendeten Charge mit 15,050 microgramm Mistellektin/ml angegeben wird. Das bedeutet eine Mistellektin-Konzentration von 7,5 nanogramm Lektine pro ml in den Inkubationsexperimenten. Die Konzentration von 7,5 nanogramm Lektine pro ml führt nach 48 h zu einer Wachstumshemmung von nahezu 20-30% in LNT-229 und SMA560 Glioma Zellen.

Auch enthält ISCADOR Q u.a. verschiedene zytotoxische Viscotoxine, deren Gehalt in der verwendeten Charge mit 364 microgramm Viscotoxin/ml angegeben wird. Das bedeutet eine Viscotoxin-Konzentration von 182 nanogramm Viscotoxine pro ml Inkubationslösung.

Bei Verwendung von ISCADOR ist somit nicht ersichtlich, auf welchen der zahlreichen Inhaltsstoffe die Wachstumshemmung der LNT-229 und SMA560 Glioma-Zellen zurückzuführen ist.

Das rekombinante Mistellektin Aviscuminum hemmt das Wachstum verschiedener Hirntumorzellen um 100% in einem Konzentrationsbereich von 0,4 - 11 ng/ml und ist als Hemmstof von Hirntumorzellen somit >10 fach potenter als ISCADOR Q. Will man die Wirkung von ISCADOR Q auf die in ihm enthaltenen Mistellektine zurückführen, muss man somit Mistellektinantagonisierende Inhaltsstoffe im fermentierten Extrakt annehmen.

Auch andere schwankende Bestandteile von Mistelextrakten sind zytotoxisch (s. Eggenschwiler J, von BL, Stritt B, Pruntsch D, Ramos M, Urech K, Rist L, Simoes-Wust AP, Viviani A; Mistletoe lectin is not the only cytotoxic component in fermented preparations of Viscum album from white fir (Abies pectinata). BMC Complement Altern Med 2007, 7:14).

## Patentansprüche

1. Arzneimittel enthaltend rekombinantes Mistellektin zur Behandlung von Hirntumor, insbesondere primäre Hirntumore, Gliome, Glioblastome, Meningeome und Hypophysenadenome, wobei das rekombinante Mistellektin ausgewählt ist aus der Gruppe der Aminosäuresequenzen SEQ ID No. 1 - 12 oder Teile und Fragmente davon umfasst oder eine Kombination davon.

2. Arzneimittel nach Anspruch 1, wobei das rekombinante Mistellektin-Polypeptid eine Mistellektin-A-Kette ist, ausgewählt aus der Gruppe der Aminosäuresequenzen SEQ ID No. 1 - 3 oder Teile und Fragmente davon umfasst oder eine Kombination davon.

3. Arzneimittel nach Anspruch 1, wobei das rekombinante Mistellektin-Polypeptid eine Mistellektin-B-Kette ist, ausgewählt aus der Gruppe der Aminosäuresequenzen SEQ ID No. 4 - 12 oder Teile und Fragmente davon umfasst oder eine Kombination davon.

4. Arzneimittel nach einem der Ansprüche 1 bis 3, wobei das Arzneimittel ausgewählt ist für Nicht-Responder und Therapieversager einer Standardtumortherapie.

5. Arzneimittel enthaltend ein rekombinantes Mistellektin-Polypeptid gemäß einem der Ansprüche 1 bis 4, gegebenenfalls zusammen mit einem pharmazeutisch verträglichen Träger.

6. Arzneimittel enthaltend ein rekombinantes Mistellektin-Polypeptid gemäß einem der Ansprüche 1 bis 5 enthaltend rekombinantes Mistellektin zur spezifischen Behandlung eines Hirntumors beim Menschen, wobei das Arzneimittel in einer Dosierung in einem Bereich von 3 - 7 ng rekombinantem Mistellektin pro kg Körpergewicht eingesetzt wird.

7. Arzneimittel nach Anspruch 6, wobei das Arzneimittel in einer Dosierung von 5 ng rekombinantem Mistellektin pro kg Körpergewicht eingesetzt wird.

8. Arzneimittel enthaltend ein rekombinantes Mistellektin-Polypeptid gemäß einem der Ansprüche 1 bis 7 enthaltend rekombinantes Mistellektin zur spezifischen Behandlung eines Hirntumors beim Menschen, wobei unabhängig vom Körpergewicht die Dosis an rekombinantem Mistellektin 200 - 500 ng beträgt.

9. Arzneimittel nach Anspruch 8, wobei unabhängig vom Körpergewicht die Dosis an rekombinantem Mistellektin 350 ng beträgt.

10. Arzneimittel nach einem oder mehreren der vorgehenden Ansprüche, wobei das Arzneimittel mindestens einmal pro Woche, vorzugsweise 2 oder 3 mal pro Woche verabreicht wird.

11. Pharmazeutische Zusammensetzung enthaltend mindestens ein rekombinantes Mistellektin gemäß einem der Ansprüche 1 bis 10, zusammen mit einem pharmazeutisch verträglichen Träger oder ggfs. weiteren Hilfs- und Zusatzstoffen.


SEQUENCE LISTING

<110>  Melema Pharma GmbH

<120>  Arzneimittel enthaltend rekombinante
        Mistellektine zur Behandlung von Hirntumoren

<130>  MEL204-02EP

<160>  12

<170>  PatentIn version 3.3

<210>  1
<211>  253
<212>  PRT
<213>  Artificial Sequence

<220>
<223>  EP0751221 recombinant Protein

<220>
<221>  misc_feature
<222>  (1)..(1)
<223>  Xaa can be Met or can be deleted

<220>
<221>  misc_feature
<222>  (5)..(5)
<223>  Xaa can be Ile or Leu

<220>
<221>  misc_feature
<222>  (16)..(16)
<223>  Xaa can be Glu or Asp

<400>  1

Xaa Tyr Glu Arg Xaa Arg Leu Arg Val Thr His Gln Thr Thr Gly Xaa
1               5                   10                  15

Glu Tyr Phe Arg Phe Ile Thr Leu Leu Arg Asp Tyr Val Ser Ser Gly
            20                  25                  30

Ser Phe Ser Asn Glu Ile Pro Leu Leu Arg Gln Ser Thr Ile Pro Val
        35                  40                  45

Ser Asp Ala Gln Arg Phe Val Leu Val Glu Leu Thr Asn Gln Gly Gly
    50                  55                  60

Asp Ser Ile Thr Ala Ala Ile Asp Val Thr Asn Leu Tyr Val Val Ala
65                  70                  75                  80

Tyr Gln Ala Gly Asp Gln Ser Tyr Phe Leu Arg Asp Ala Pro Arg Gly
                85                  90                  95

Ala Glu Thr His Leu Phe Thr Gly Thr Thr Arg Ser Ser Leu Pro Phe
                100                 105                 110

Asn Gly Ser Tyr Pro Asp Leu Glu Arg Tyr Ala Gly His Arg Asp Gln
        115                 120                 125

Ile Pro Leu Gly Ile Asp Gln Leu Ile Gln Ser Val Thr Ala Leu Arg
    130                 135                 140

Phe Pro Gly Gly Ser Thr Arg Thr Gln Ala Arg Ser Ile Leu Ile Leu
145                 150                 155                 160

Ile Gln Met Ile Ser Glu Ala Ala Arg Phe Asn Pro Ile Leu Trp Arg
                165                 170                 175

Ala Arg Gln Tyr Ile Asn Ser Gly Ala Ser Phe Leu Pro Asp Val Tyr
                180                 185                 190

Met Leu Glu Leu Glu Thr Ser Trp Gly Gln Gln Ser Thr Gln Val Gln
        195                 200                 205

His Ser Thr Asp Gly Val Phe Asn Asn Pro Ile Arg Leu Ala Ile Pro
    210                 215                 220

Pro Gly Asn Phe Val Thr Leu Thr Asn Val Arg Asp Val Ile Ala Ser
225                 230                 235                 240

Leu Ala Ile Met Leu Phe Val Cys Gly Glu Arg Pro Ser
                245                 250

<210>  2
<211>  257
<212>  PRT
<213>  Artificial Sequence

<220>
<223>  EP1051495

<220>
<221>  misc_feature
<222>  (1)..(1)
<223>  Xaa can be Met or can be deleted

<220>
<221>  misc_feature
<222>  (16)..(16)
<223>  Xaa can be Asp or Glu

<220>
<221>  misc_feature
<222>  (64)..(64)
<223>  Xaa can be Gly or Gln

<220>
<221>  misc_feature
<222>  (67)..(67)
<223>  Xaa can be Ile or Val

<220>
<221>  misc_feature
<222>  (76)..(76)
<223>  Xaa can be Leu or Ala

<220>
<221>  misc_feature
<222>  (108)..(109)
<223>  Xaa can be Asp-Arg or can be deleted

<220>
<221>  misc_feature
<222>  (115)..(115)
<223>  Xaa can be Asn or Thr

<220>
<221>  misc_feature
<222>  (119)..(119)
<223>  Xaa can be Pro or Thr

<220>
<221>  misc_feature
<222>  (136)..(136)
<223>  Xaa can be Asp or Glu

<220>
<221>  misc_feature
<222>  (143)..(143)
<223>  Xaa can be Ser or Thr

<220>
<221>  misc_feature
<222>  (147)..(147)
<223>  Xaa can be Phe or Tyr

<220>
<221>  misc_feature
<222>  (154)..(154)
<223>  Xaa can be Ala or Thr

<220>
<221>  misc_feature
<222>  (179)..(179)
<223>  Xaa can be Ala or Tyr

<220>
<221>  misc_feature
<222>  (182)..(182)
<223>  Xaa can be Tyr or Asp

<220>
<221>  misc_feature
<222>  (187)..(187)
<223>  Xaa can be Ala or Glu

<220>
<221>  misc_feature

<222>  (193)..(193)
<223>  Xaa can be Val or Met

<220>
<221>  misc_feature
<222>  (221)..(221)
<223>  Xaa can be Ile or Phe

<220>
<221>  misc_feature
<222>  (227)..(227)
<223>  Xaa can be Ser or Thr

<220>
<221>  misc_feature
<222>  (234)..(234)
<223>  Xaa can be Thr or Ser

<220>
<221>  misc_feature
<222>  (238)..(238)
<223>  Xaa can be Asp or Ser

<220>
<221>  misc_feature
<222>  (256)..(257)
<223>  Xaa can be Ser-Ser or can be deleted

<400>  2

Xaa Tyr Glu Arg Leu Arg Leu Arg Val Thr His Gln Thr Thr Gly Xaa
1               5                   10                  15

Glu Tyr Phe Arg Phe Ile Thr Leu Leu Arg Asp Tyr Val Ser Ser Gly
            20                  25                  30

Ser Phe Ser Asn Glu Ile Pro Leu Leu Arg Gln Ser Thr Ile Pro Val
        35                  40                  45

Ser Asp Ala Gln Arg Phe Val Leu Val Glu Leu Thr Asn Gln Gly Xaa
    50                  55                  60

Asp Ser Xaa Thr Ala Ala Ile Asp Val Thr Asn Xaa Tyr Val Val Ala
65                  70                  75                  80

Tyr Gln Ala Gly Asp Gln Ser Tyr Phe Leu Arg Asp Ala Pro Arg Gly
                85                  90                  95

Ala Glu Thr His Leu Phe Thr Gly Thr Thr Arg Xaa Xaa Ser Ser Leu
            100                 105                 110

Pro Phe Xaa Gly Ser Tyr Xaa Asp Leu Glu Arg Tyr Ala Gly His Arg
        115                 120                 125

Asp Gln Ile Pro Leu Gly Ile Xaa Gln Leu Ile Gln Ser Val Xaa Ala

    130                 135                 140

Leu Arg Xaa Pro Gly Gly Ser Thr Arg Xaa Gln Ala Arg Ser Ile Leu
145                 150                 155                 160

Ile Leu Ile Gln Met Ile Ser Glu Ala Ala Arg Phe Asn Pro Ile Leu
                165                 170                 175

Trp Arg Xaa Arg Gln Xaa Ile Asn Ser Gly Xaa Ser Phe Leu Pro Asp
            180                 185                 190

Xaa Tyr Met Leu Glu Leu Glu Thr Ser Trp Gly Gln Gln Ser Thr Gln
        195                 200                 205

Val Gln His Ser Thr Asp Gly Val Phe Asn Asn Pro Xaa Arg Leu Ala
    210                 215                 220

Ile Pro Xaa Gly Asn Phe Val Thr Leu Xaa Asn Val Arg Xaa Val Ile
225                 230                 235                 240

Ala Ser Leu Ala Ile Met Leu Phe Val Cys Gly Glu Arg Pro Ser Xaa
                245                 250                 255

Xaa

<210> 3
<211> 257
<212> PRT
<213> Artificial Sequence

<220>
<223> EP1051495

<220>
<221> misc_feature
<222> (1)..(1)
<223> Xaa can be Met or can be deleted

<400> 3

Xaa Tyr Glu Arg Leu Arg Leu Arg Val Thr His Gln Thr Thr Gly Asp
1               5                   10                  15

Glu Tyr Phe Arg Phe Ile Thr Leu Leu Arg Asp Tyr Val Ser Ser Gly
            20                  25                  30

Ser Phe Ser Asn Glu Ile Pro Leu Leu Arg Gln Ser Thr Ile Pro Val
        35                  40                  45

Ser Asp Ala Gln Arg Phe Val Leu Val Glu Leu Thr Asn Gln Gly Gln
    50                  55                  60

Asp Ser Ile Thr Ala Ala Ile Asp Val Thr Asn Ala Tyr Val Val Ala
65                  70                  75                  80

Tyr Gln Ala Gly Asp Gln Ser Tyr Phe Leu Arg Asp Ala Pro Arg Gly
                85                  90                  95

Ala Glu Thr His Leu Phe Thr Gly Thr Thr Arg Asp Arg Ser Ser Leu
            100                 105                 110

Pro Phe Thr Gly Ser Tyr Thr Asp Leu Glu Arg Tyr Ala Gly His Arg
        115                 120                 125

Asp Gln Ile Pro Leu Gly Ile Glu Gln Leu Ile Gln Ser Val Ser Ala
    130                 135                 140

Leu Arg Tyr Pro Gly Gly Ser Thr Arg Ala Gln Ala Arg Ser Ile Leu
145                 150                 155                 160

Ile Leu Ile Gln Met Ile Ser Glu Ala Ala Arg Phe Asn Pro Ile Leu
                165                 170                 175

Trp Arg Tyr Arg Gln Asp Ile Asn Ser Gly Glu Ser Phe Leu Pro Asp
            180                 185                 190

Met Tyr Met Leu Glu Leu Glu Thr Ser Trp Gly Gln Gln Ser Thr Gln
        195                 200                 205

Val Gln His Ser Thr Asp Gly Val Phe Asn Asn Pro Phe Arg Leu Ala
    210                 215                 220

Ile Ser Thr Gly Asn Phe Val Thr Leu Ser Asn Val Arg Ser Val Ile
225                 230                 235                 240

Ala Ser Leu Ala Ile Met Leu Phe Val Cys Gly Glu Arg Pro Ser Ser
                245                 250                 255

Ser

<210>  4
<211>  264
<212>  PRT
<213>  Artificial Sequence

<220>
<223>  EP0751221

<220>
<221>  misc_feature
<222>  (1)..(1)
<223>  Xaa can be Met or can be deleted

<400>  4

Xaa Asp Asp Val Thr Cys Ser Ala Ser Glu Pro Thr Val Arg Ile Val
1               5                   10                  15

Gly Arg Asn Gly Met Cys Val Asp Val Arg Asp Asp Asp Phe Arg Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Gly Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Leu Trp Gln Ile Trp Gly
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gly Gly Ser
145                 150                 155                 160

Val Trp Val Glu Thr Cys Val Ser Ser Gln Lys Asn Gln Arg Trp Ala
                165                 170                 175

Leu Tyr Gly Asp Gly Ser Ile Arg Pro Lys Gln Asn Gln Asp Gln Cys
            180                 185                 190

Leu Thr Cys Gly Arg Asp Ser Val Ser Thr Val Ile Asn Ile Val Ser
        195                 200                 205

Cys Ser Ala Gly Ser Ser Gly Gln Arg Trp Val Phe Thr Asn Glu Gly
    210                 215                 220

Ala Ile Leu Asn Leu Lys Asn Gly Leu Ala Met Asp Val Ala Gln Ala
225                 230                 235                 240

Asn Pro Lys Leu Arg Arg Ile Ile Ile Tyr Pro Ala Thr Gly Lys Pro
                245                 250                 255

Asn Gln Met Trp Leu Pro Val Pro
            260

<210>  5
<211>  268
<212>  PRT
<213>  Artificial Sequence

<220>
<223>  EP0751221

<220>
<221>  misc_feature
<222>  (1)..(1)
<223>  Xaa can be Met or can be deleted

<400>  5

Xaa Asp Asp Val Thr Cys Ser Ala Ser Glu Pro Thr Val Arg Ile Val
1               5                   10                  15

Gly Arg Asn Gly Met Cys Val Asp Val Arg Asp Asp Asp Phe Arg Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Gly Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Leu Trp Gln Ile Trp Gly
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val

130                 135                 140

Thr Ile Tyr Gly Phe Arg Asp Leu Cys Met Glu Ser Asn Gly Gly Ser
145                 150                 155                 160

Val Trp Val Glu Thr Cys Val Ser Ser Gln Lys Asn Gln Arg Trp Ala
                165                 170                 175

Leu Tyr Gly Asp Gly Ser Ile Arg Pro Lys Gln Asn Gln Asp Gln Cys
            180                 185                 190

Leu Thr Cys Gly Arg Asp Ser Val Ser Thr Val Ile Asn Ile Val Ser
        195                 200                 205

Cys Ser Ala Gly Ser Ser Gly Gln Arg Trp Val Phe Thr Asn Glu Gly
    210                 215                 220

Ala Ile Leu Asn Leu Lys Asn Gly Leu Ala Met Asp Val Ala Gln Ala
225                 230                 235                 240

Asn Pro Lys Leu Arg Arg Ile Ile Ile Tyr Pro Ala Thr Gly Lys Pro
                245                 250                 255

Asn Gln Met Trp Leu Pro Val Pro Gly Gly Tyr His
            260                 265

<210>  6
<211>  265
<212>  PRT
<213>  Artificial

<220>
<223>  EP1051495 recombinant Protein


<220>
<221>  misc_feature
<222>  (1)..(1)
<223>  Xaa can be Met or can be deleted

<220>
<221>  misc_feature
<222>  (19)..(19)
<223>  Xaa can be Asn or Ser

<220>
<221>  misc_feature
<222>  (22)..(22)
<223>  Xaa can be Cys or Arg

<220>
<221>  misc_feature
<222>  (57)..(57)
<223>  Xaa can be Gly or Asn

<220>
<221>  misc_feature
<222>  (96)..(96)
<223>  Xaa can be Gly or Asn

<220>
<221>  misc_feature
<222>  (158)..(158)
<223>  Xaa can be Gly or Gln
<220>
<221>  misc_feature
<222>  (167)..(167)
<223>  Xaa can be Val or Asp

<220>
<221>  misc_feature
<222>  (171)..(171)
<223>  Xaa can be Gln or Lys

<220>
<221>  misc_feature
<222>  (174)..(175)
<223>  Xaa is Gly, Gly-Arg, Gly-Lys, Arg, Lys or can be deleted

<220>
<221>  misc_feature
<222>  (196)..(196)
<223>  Xaa can be Cys or Val or Ser

<220>
<221>  misc_feature
<222>  (212)..(213)
<223>  Xaa can be Ala-Ala or Ala-Gly or Gly-Ala or Gly-Gly

<220>
<221>  misc_feature
<222>  (215)..(216)
<223>  Xaa can be Ser-Ser or Ser-Gly or Gly-Ser or Gly-Gly

<220>
<221>  misc_feature
<222>  (225)..(225)
<223>  Xaa can be Gly or Tyr

<220>
<221>  misc_feature
<222>  (232)..(236)
<223>  232Asn,Ser,Thr,Lys;233Ser,Gly;234Leu,Pro;235Ala,Met;236:Met,Val

<220>
<221>  misc_feature
<222>  (265)..(265)
<223>  Xaa can be Pro or Phe

<400>  6

Xaa Asp Asp Val Thr Cys Ser Ala Ser Glu Pro Thr Val Arg Ile Val
1               5                   10                  15


Gly Arg Xaa Gly Met Xaa Val Asp Val Arg Asp Asp Asp Phe His Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Xaa Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Ile Trp Gln Ile Trp Xaa
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Xaa Gly Ser
145                 150                 155                 160

Val Trp Val Glu Thr Cys Xaa Ser Ser Gln Xaa Asn Gln Xaa Xaa Trp
                165                 170                 175

Ala Leu Tyr Gly Asp Gly Ser Ile Arg Pro Lys Gln Asn Gln Asp Gln
            180                 185                 190

Cys Leu Thr Xaa Gly Arg Asp Ser Val Ser Thr Val Ile Asn Ile Val
        195                 200                 205

Ser Cys Ser Xaa Xaa Ser Xaa Xaa Gln Arg Trp Val Phe Thr Asn Glu
    210                 215                 220

Xaa Ala Ile Leu Asn Leu Lys Xaa Xaa Xaa Xaa Xaa Asp Val Ala Gln
225                 230                 235                 240

Ala Asn Pro Lys Leu Arg Arg Ile Ile Ile Tyr Pro Ala Thr Gly Lys
                245                 250                 255

Pro Asn Gln Met Trp Leu Pro Val Xaa
            260                 265

<210>  7

<211>  264
<212>  PRT
<213>  Artificial

<220>
<223>  EP1051495 recombinant Protein


<220>
<221>  misc_feature
<222>  (1)..(1)
<223>  Xaa can be Met or can be deleted

<400>  7

Xaa Asp Asp Val Thr Cys Ser Ala Ser Glu Pro Thr Val Arg Ile Val
1               5                   10                  15

Gly Arg Asn Gly Met Cys Val Asp Val Arg Asp Asp Asp Phe His Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Gly Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Ile Trp Gln Ile Trp Gly
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gly Gly Ser
145                 150                 155                 160

Val Trp Val Glu Thr Cys Val Ser Ser Gln Gln Asn Gln Arg Trp Ala
                165                 170                 175

Leu Tyr Gly Asp Gly Ser Ile Arg Pro Lys Gln Asn Gln Asp Gln Cys
            180                 185                 190

Leu Thr Cys Gly Arg Asp Ser Val Ser Thr Val Ile Asn Ile Val Ser
        195                 200                 205

Cys Ser Ala Gly Ser Ser Gly Gln Arg Trp Val Phe Thr Asn Glu Gly
    210                 215                 220

Ala Ile Leu Asn Leu Lys Asn Gly Leu Ala Met Asp Val Ala Gln Ala
225                 230                 235                 240

Asn Pro Lys Leu Arg Arg Ile Ile Ile Tyr Pro Ala Thr Gly Lys Pro
                245                 250                 255

Asn Gln Met Trp Leu Pro Val Pro
            260

<210>  8
<211>  265
<212>  PRT
<213>  Artificial

<220>
<223>  EP1051495 recombinant Protein

<220>
<221>  misc_feature
<222>  (1)..(1)
<223>  Xaa can be Met or can be deleted

<400>  8

Xaa Asp Asp Val Thr Cys Ser Ala Ser Glu Pro Thr Val Arg Ile Val
1               5                   10                  15

Gly Arg Asn Gly Met Arg Val Asp Val Arg Asp Asp Asp Phe His Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Gly Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Ile Trp Gln Ile Trp Asp
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gly Gly Ser
145                 150                 155                 160

Val Trp Val Glu Thr Cys Asp Ser Ser Gln Lys Asn Gln Gly Lys Trp
                165                 170                 175

Ala Leu Tyr Gly Asp Gly Ser Ile Arg Pro Lys Gln Asn Gln Asp Gln
            180                 185                 190

Cys Leu Thr Ser Gly Arg Asp Ser Val Ser Thr Val Ile Asn Ile Val
        195                 200                 205

Ser Cys Ser Gly Ala Ser Gly Ser Gln Arg Trp Val Phe Thr Asn Glu
    210                 215                 220

Gly Ala Ile Leu Asn Leu Lys Asn Gly Leu Ala Met Asp Val Ala Gln
225                 230                 235                 240

Ala Asn Pro Lys Leu Arg Arg Ile Ile Ile Tyr Pro Ala Thr Gly Lys
                245                 250                 255

Pro Asn Gln Met Trp Leu Pro Val Phe
            260                 265

<210>  9
<211>  265
<212>  PRT
<213>  Artificial

<220>
<223>  EP1051495 recombinant Protein

<220>
<221>  misc_feature
<222>  (1)..(1)
<223>  Xaa can be Met or can be deleted

<400>  9

Xaa Asp Asp Val Thr Cys Ser Ala Ser Glu Pro Thr Val Arg Ile Val
1               5                   10                  15

Gly Arg Ser Gly Met Arg Val Asp Val Arg Asp Asp Asp Phe His Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Asn Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Ile Trp Gln Ile Trp Asp
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gln Gly Ser
145                 150                 155                 160

Val Trp Val Glu Thr Cys Asp Ser Ser Gln Lys Asn Gln Gly Lys Trp
                165                 170                 175

Ala Leu Tyr Gly Asp Gly Ser Ile Arg Pro Lys Gln Asn Gln Asp Gln
            180                 185                 190

Cys Leu Thr Val Gly Arg Asp Ser Val Ser Thr Val Ile Asn Ile Val
        195                 200                 205

Ser Cys Ser Gly Ala Ser Gly Ser Gln Arg Trp Val Phe Thr Asn Glu
    210                 215                 220

Tyr Ala Ile Leu Asn Leu Lys Ser Gly Leu Ala Met Asp Val Ala Gln
225                 230                 235                 240

Ala Asn Pro Lys Leu Arg Arg Ile Ile Ile Tyr Pro Ala Thr Gly Lys
                245                 250                 255

Pro Asn Gln Met Trp Leu Pro Val Phe
            260                 265

<210>  10

<211>  265
<212>  PRT
<213>  Artificial

<220>
<223>  EP1051495 recombinant Protein


<220>
<221>  misc_feature
<222>  (1)..(1)
<223>  Xaa can be Met or can be deleted

<400>  10

Xaa Asp Asp Val Thr Cys Ser Ala Ser Glu Pro Thr Val Arg Ile Val
1               5                   10                  15


Gly Arg Asn Gly Met Arg Val Asp Val Arg Asp Asp Asp Phe His Asp
            20                  25                  30


Gly Asn Gln Ile Gln Leu Trp Pro Ser Lys Ser Asn Asn Asp Pro Asn
        35                  40                  45


Gln Leu Trp Thr Ile Lys Arg Asp Gly Thr Ile Arg Ser Asn Gly Ser
    50                  55                  60


Cys Leu Thr Thr Tyr Gly Tyr Thr Ala Gly Val Tyr Val Met Ile Phe
65                  70                  75                  80


Asp Cys Asn Thr Ala Val Arg Glu Ala Thr Ile Trp Gln Ile Trp Asp
                85                  90                  95


Asn Gly Thr Ile Ile Asn Pro Arg Ser Asn Leu Val Leu Ala Ala Ser
            100                 105                 110


Ser Gly Ile Lys Gly Thr Thr Leu Thr Val Gln Thr Leu Asp Tyr Thr
        115                 120                 125


Leu Gly Gln Gly Trp Leu Ala Gly Asn Asp Thr Ala Pro Arg Glu Val
    130                 135                 140


Thr Ile Tyr Gly Phe Arg Asp Leu Cys Met Glu Ser Asn Gly Gly Ser
145                 150                 155                 160


Val Trp Val Glu Thr Cys Asp Ser Ser Gln Lys Asn Gln Gly Lys Trp
                165                 170                 175


Ala Leu Tyr Gly Asp Gly Ser Ile Arg Pro Lys Gln Asn Gln Asp Gln
            180                 185                 190

Cys Leu Thr Ser Gly Arg Asp Ser Val Ser Thr Val Ile Asn Ile Val
        195                 200                 205

Ser Cys Ser Gly Ala Ser Gly Ser Gln Arg Trp Val Phe Thr Asn Glu
    210                 215                 220

Gly Ala Ile Leu Asn Leu Lys Thr Gly Leu Ala Met Asp Val Ala Gln
225                 230                 235                 240

Ala Asn Pro Lys Leu Arg Arg Ile Ile Ile Tyr Pro Ala Thr Gly Lys
                245                 250                 255

Pro Asn Gln Met Trp Leu Pro Val Phe
            260                 265

<210>  11
<211>  265
<212>  PRT
<213>  Artificial

<220>
<223>  EP1051495 recombinant Protein

<220>
<221>  misc_feature
<222>  (1)..(1)
<223>  Xaa can be Met or can be deleted

<400>  11

Xaa Asp Asp Val Thr Cys Ser Ala Ser Glu Pro Thr Val Arg Ile Val
1               5                   10                  15

Gly Arg Asn Gly Met Arg Val Asp Val Arg Asp Asp Asp Phe His Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Gly Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Ile Trp Gln Ile Trp Asp
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gly Gly Ser
145                 150                 155                 160

Val Trp Val Glu Thr Cys Asp Ser Ser Gln Lys Asn Gln Gly Lys Trp
                165                 170                 175

Ala Leu Tyr Gly Asp Gly Ser Ile Arg Pro Lys Gln Asn Gln Asp Gln
            180                 185                 190

Cys Leu Thr Ser Gly Arg Asp Ser Val Ser Thr Val Ile Asn Ile Val
        195                 200                 205

Ser Cys Ser Gly Ala Ser Gly Ser Gln Arg Trp Val Phe Thr Asn Glu
    210                 215                 220

Gly Ala Ile Leu Asn Leu Lys Lys Gly Pro Ala Met Asp Val Ala Gln
225                 230                 235                 240

Ala Asn Pro Lys Leu Arg Arg Ile Ile Ile Tyr Pro Ala Thr Gly Lys
                245                 250                 255

Pro Asn Gln Met Trp Leu Pro Val Phe
            260                 265

<210>  12
<211>  265
<212>  PRT
<213>  Artificial

<220>
<223>  EP1051495 recombinant Protein

<220>
<221>  misc_feature
<222>  (1)..(1)
<223>  Xaa can be Met or can be deleted

<400>  12

Xaa Asp Asp Val Thr Cys Ser Ala Ser Glu Pro Thr Val Arg Ile Val
1               5                   10                  15

Gly Arg Asn Gly Met Arg Val Asp Val Arg Asp Asp Asp Phe His Asp
            20                  25                  30

Gly Asn Gln Ile Gln Leu Trp Pro Ser Lys Ser Asn Asn Asp Pro Asn
        35                  40                  45

Gln Leu Trp Thr Ile Lys Arg Asp Gly Thr Ile Arg Ser Asn Gly Ser
    50                  55                  60

Cys Leu Thr Thr Tyr Gly Tyr Thr Ala Gly Val Tyr Val Met Ile Phe
65                  70                  75                  80

Asp Cys Asn Thr Ala Val Arg Glu Ala Thr Ile Trp Gln Ile Trp Asp
                85                  90                  95

Asn Gly Thr Ile Ile Asn Pro Arg Ser Asn Leu Val Leu Ala Ala Ser
            100                 105                 110

Ser Gly Ile Lys Gly Thr Thr Leu Thr Val Gln Thr Leu Asp Tyr Thr
        115                 120                 125

Leu Gly Gln Gly Trp Leu Ala Gly Asn Asp Thr Ala Pro Arg Glu Val
    130                 135                 140

Thr Ile Tyr Gly Phe Arg Asp Leu Cys Met Glu Ser Asn Gly Gly Ser
145                 150                 155                 160

Val Trp Val Glu Thr Cys Asp Ser Ser Gln Lys Asn Gln Gly Lys Trp
                165                 170                 175

Ala Leu Tyr Gly Asp Gly Ser Ile Arg Pro Lys Gln Asn Gln Asp Gln
            180                 185                 190

Cys Leu Thr Ser Gly Arg Asp Ser Val Ser Thr Val Ile Asn Ile Val
        195                 200                 205

Ser Cys Ser Gly Ala Ser Gly Ser Gln Arg Trp Val Phe Thr Asn Glu
    210                 215                 220

Gly Ala Ile Leu Asn Leu Lys Asn Ser Leu Met Val Asp Val Ala Gln
225                 230                 235                 240

Ala Asn Pro Lys Leu Arg Arg Ile Ile Ile Tyr Pro Ala Thr Gly Lys
                245                 250                 255

Pro Asn Gln Met Trp Leu Pro Val Phe
            260                 265